# EUROPÄISCHE PATENTSCHRIFT

(11) **EP 0 367 087 B1**
(45) Veröffentlichungstag und Bekanntmachung des Hinweises auf die Patenterteilung: **18.01.1995**
(21) Anmeldenummer: 89119802.0
(22) Anmeldetag: 25.10.1989
(51) Int. Cl.: C08G 65/32, C08G 65/20, C07C 43/16, C07C 41/08

(54) **Polytetrahydrofuranvinylether und ihre Herstellung**
Politetrahydrofuranvinylether and their preparation
Vinyléthers de polytétrahydrofurane et leur préparation

(30) Priorität: 04.11.1988 DE 3837469
(43) Veröffentlichungstag der Anmeldung: 09.05.1990
(73) Patentinhaber: BASF Aktiengesellschaft, 67063 Ludwigshafen (DE)
(72) Erfinder: Karcher, Michael, Dr., D-6915 Dossenheim (DE); Eckhardt, Heinz, Dr., D-6700 Ludwigshafen (DE); Henkelmann, Jochem, Dr., D-6140 Bensheim (DE)

(56) Entgegenhaltungen:
- EP-A- 0 066 179
- US-A- 2 066 076
- US-A- 4 751 273
- US-A- 4 766 252
- JOURNAL OF MACROMOLECULAR SCIENCE. REVIEWS IN MACROMOLECULAR CHEMISTRY AND PHYSICS Band C25, Nr. 3, 1985, Seiten 325-373, New York, USA; J. MILTON HARRIS: "Laboratory synthesis of Polyethylene Glycol Derivaties" *Seite 334, obere Gleichung *

## Beschreibung

Diese Erfindung betrifft neue Polytetrahydrofuranvinylether sowie ein Verfahren zu ihrer Herstellung.

Polytetrahydrofuran und Derivate des Polytetrahydrofurans haben als Zwischenprodukte für die Herstellung von Polymeren großes Interesse gefunden. Besonders gesucht sind hierbei Polytetrahydrofuranderivate mit endständigen reaktiven Gruppen wie Polytetrahydrofuran-ω,ω'-diamine (US-A-3,824,198), die den Einbau der Polybutandiol-Kette in Polymere ermöglichen.

US-A-4 751 273 betrifft Stoffzusammensetzungen aus Vinylether-Endgruppen-haltigen Urethanharzen, deren Herstellung sowie die Härtung dieser Stoffzusammensetzungen zu festen, nicht klebenden Lacküberzügen. Zu diesem Zweck werden verschiedene Glykole einer Reppe-Vinylierung unterworfen, wobei ein Gemisch aus dem Mono- und Divinylether des betreffenden Glykols gebildet wird, das zusätzlich noch das betreffende Glykol enthält.

Gegenstand dieser Erfindung sind Polytetrahydrofuranderivate, die sich vorteilhaft zum Aufbau polymerer Kunststoffe eignen. Diese Verbindungen haben die allgemeine Formel

H₂C=HC⁅O-CH₂-CH₂-CH₂-CH₂⁆ₙO-CH=CH₂ I,

in der n eine Zahl von 3 bis 70 und insbesondere 3 bis 55 bedeutet.

Man stellt die Polytetrahydrofuranvinylether der Formel I dadurch her, daß man Polytetrahydrofuran der Formel

H⁅O-CH₂-CH₂-CH₂-CH₂⁆ₙOH II

in der n die obengenannte Bedeutung hat, mit mindestens der stöchiometrischen Menge an Acetylen in Gegenwart von Vinylierungskatalysatoren bei Temperaturen von 100 bis 200°C und Drücken von 5 bis 25 atm umsetzt.

Die Umsetzung von Alkoholen mit Acetylen in Gegenwart stark basischer Katalysatoren ist als Vinylierungsreaktion nach Reppe (s. Liebigs Annalen der Chemie 601, 81 (1956) bekannt. Wendet man die Vinylierungsreaktion auf längerkettige mehrwertige Alkohole an, so ist damit zu rechnen, daß der primär entstehende Monovinylether mit einer freien Hydroxylgruppe des gleichen Moleküls unter Bildung eines cyclischen Acetaldehydacetals reagiert. Es war deshalb überraschend, daß bei der erfindungsgemäßen Vinylierung ausschließlich die vinylierten Produkte der Formel I erhalten werden.

Man führt die Vinylierung gemäß der Erfindung bei Temperaturen von 100 bis 200, vorzugsweise 150 bis 170°C und Drücken von 5 bis 25, vorzugsweise 10 bis 25 atm durch. Als Katalysatoren werden an sich bekannte Vinylierungskatalysatoren, insbesondere stark basische Katalysatoren, wie Alkalihydroxide, z.B. KOH oder NaOH oder Alkalialkoholate, wie Natriummethanolat, Natriumethanolat, Kaliummethanolat, Kalium-tert.butanolat eingesetzt. Die Katalysatoren werden z.B. in Mengen von 1 bis 10 Gew.%, bezogen auf das Polytetrahydrofuran, angewendet.

Acetylen wird aus Sicherheitsgründen bevorzugt mit inerten Gasen, wie Stickstoff, verdünnt. Die Vinylierung wird in Abwesenheit oder Anwesenheit von Lösungsmitteln durchgeführt. Als Lösungsmittel sind z.B. Tetrahydrofuran, Dioxan, Toluol, Decalin, Cyclohexan oder N-Methyl-pyrrolidon geeignet. Um eine vollständige Vinylierung der Ausgangsstoffe zu den Divinylethern zu erzielen, wendet man mindestens die stöchiometrische Menge Acetylen an.

### Beispiel 1

3 kg (12 mol) Polytetrahydrofuran mit einem Molekulargewicht von 250 (n = 3,22) werden in einem 8 l Autoklaven mit 60 g (1,07 mol) KOH-Pulver versetzt. Der Autoklav wird mit Stickstoff gespült und auf 160°C erhitzt. Nach Erreichen der Reaktionstemperatur (160°C) werden zuerst 10 atm Stickstoff und danach 10 atm Acetylen aufgepreßt. In dem Maße, in dem das Acetylen verbraucht wird, wird Acetylen zugegeben. Nach Reaktionsende (etwa 24 h) wird der Austrag destilliert. Bei einer maximalen Übergangstemperatur von 155°C bei 0,3 bar erhält man 3,3 kg vinyliertes Polytetrahydrofuran mit einem Anteil an Polytetrahydrofurandivinylether (s. Formel I mit n = 3,22) von >95 Gew.%.

### Analyse:

1H-NMR(CDCl₃, 250 MHz): δ= 1.6 (m, 14.2H), 3.4 (m, 9.8H) 3.7 (t,4H), 4.0 (m, 2H), 4.2 (m, 2H), 6.4 (dd, 2H), OH-Zahl 12.

## Patentansprüche

1. Polytetrahydrofuranvinylether der allgemeinen Formel
H₂C=HC⁅O-CH₂-CH₂-CH₂-CH₂⁆ₙO-CH=CH₂ I
in der n eine Zahl von 3 bis 70 bedeutet.

2. Verfahren zur Herstellung von Polytetrahydrofurandivinylethern nach Anspruch 1, dadurch gekennzeichnet, daß man Polytetrahydrofuran der allgemeinen Formel
H⁅O-CH₂-CH₂-CH₂-CH₂⁆ₙOH II
in der n die in Anspruch 1 genannte Bedeutung hat, mit mindestens der stöchiometrischen Menge an Acetylen in Gegenwart von Vinylierungskatalysatoren bei Temperaturen von 100 bis 200°C und Drücken von 5 bis 25 atm umsetzt.

3. Verfahren nach Anspruch 2, dadurch gekennzeichnet, daß man die Umsetzung des Tetrahydrofurans mit Acetylen in Gegenwart eines Alkalihydroxids oder eines Alkalialkoholats als Katalysator durchführt.

## Claims

1. A polytetrahydrofuran divinyl ether of the formula
H₂C=HC⁅O-CH₂-CH₂-CH₂-CH₂⁆ₙO-CH=CH₂
where n is from 3 to 70.

2. A process for the preparation of a polytetrahydrofuran divinyl ether as claimed in claim 1, wherein a polytetrahydrofuran of the formula
H⁅O-CH₂-CH₂-CH₂-CH₂⁆ₙOH II
where n has the meaning stated in claim 1, is reacted with not less than the stoichiometric amount of acetylene in the presence of a vinylation catalyst at from 100 to 200°C and under from 5 to 25 atm.

3. A process as claimed in claim 2, wherein the reaction of the tetrahydrofuran with acetylene is carried out in the presence of an alkali metal hydroxide or of an alkali metal alcoholate as the catalyst.

## Revendications

1. Polytétrahydrofurannevinyléthers de la formule générale :
H₂C=HC⁅O-CH₂-CH₂-CH₂-CH₂⁆ₙO-CH=CH₂ I
dans laquelle n représente un nombre dont la valeur varie de 3 à 70.

2. Procédé de préparation de polytétrahydrofurannedivinyléthers selon la formule I, caractérisé en ce que l'on fait réagir le polytétrahydrofuranne de la formule générale :
H⁅O-CH₂-CH₂-CH₂-CH₂⁆ₙOH II
dans laquelle n a les significations qui lui ont été attribuées dans la revendication 1, avec au moins la proportion stoechiométrique d'acétylène, en présence de catalyseurs de vinylation, à des températures de 100 à 200°C et sous des pressions de 5 à 25 atm.

3. Procédé selon la revendication 2, caractérisé en ce que l'on entreprend la réaction du tétrahydrofuranne avec l'acétylène en présence d'un hydroxyde de métal alcalin ou d'un alcoolate de métal alcalin servant de catalyseur.
